# EUROPEAN PATENT SPECIFICATION

(11) **EP 2 105 149 B1**
(45) Date of publication and mention of the grant of the patent: **23.11.2011**
(21) Application number: 09156289.2
(22) Date of filing: 26.03.2009
(51) Int. Cl.: A61L 27/36, A61L 27/48

(54) **A human bone paste, its preparation and use**
Bereitung und Anwendung von Knochenpaste
Préparation et utilisation d'un ciment osseux

(30) Priority: 27.03.2008 IT FI20080058
(43) Date of publication of application: 30.09.2009
(73) Proprietor: Tissuelab S.p.A., 80029 S. Antimo (IT)
(72) Inventor: Ramelli, Massimiliano, 81100, CASERTA (IT); Della Valle, Elena, 81100, CASERTA (IT); Mariniello, Renato, 80029, S. ANTIMO (IT)
(74) Representative: Gervasi, Gemma

(56) References cited:
- WO-A1-2007/056671
- WO-A2-2007/084725
- DE-A1- 19 849 984
- US-A- 5 073 373

## Description

### Field of the invention

The present invention refers to the field of products having osteo-inductive properties.

### State of the art

Advances in surgery have led to an increase in the use of grafts, or bone tissue implants, for filling or supporting purposes, in bone repair and reconstruction surgery. In reconstruction procedures, where a loss of bone matter is involved, the orthopaedic surgeon can choose the most suitable and effective material for a given purpose, from among various types of bone and its derivatives.

Generally speaking, rather than using autologous bone tissue, it is preferable to use homologous tissue harvested from a living or cadaveric donor, distributed by accredited tissue banks.

A particular type of bone graft is the so-called demineralised bone matrix (DBM), described for the first time by Senn in 1889. This consists of the protein part of bone, deprived of its mineral component; the demineralisation process exposes the osteo-inductive factors, such as the bone morphogenetic proteins (BMP), to the surrounding tissues, thereby inducing osseous neogenesis. One of the drawbacks associated with the use of DBM, however, lies in that the dimensions of the constituent granules is such that it becomes difficult for them to remain in the area into which they are transplanted and they tend to migrate; in addition, they have a rough surface texture that makes them difficult to handle during the surgical procedure.

As a consequence, one of the objects of research in recent years has been to obtain a product, such as a bone tissue paste, that is not susceptible to migration and that is easy to use in the operating room.

Bone pastes are a particular type of bone substitute and various preparations are currently available that are distinguishable by their formulation; the main component of these preparations, however, can be either homologous bone tissue or ceramics.

Ceramics are inorganic materials that provide a matrix for the formation of new bone tissue at the site of a surgical procedure. These inorganic materials include coralline hydroxyapatite, synthetic hydroxyapatite, and tricalcium phosphate; they resemble natural bone hydroxyapatite and are considered valid osteo-conductive matrices, especially when used as fillers.

Since ceramics alone are difficult to handle during surgical procedures, the forms that are found on the market are generally mixed with substances that lend them a pasty consistency and make them easier to handle, i.e. more ductile and more adaptable to the site of the graft (C.P. Desilets, et al. "Development of synthetic bone-repair materials for craniofacial reconstruction" J. Craniofacial Surgery, Vol. 1 (3), 1990, pages 150-153).

The inorganic materials forming the pastes have no osteo-inductive properties, but they are osteo-conductive, since they have the capacity to facilitate the diffusion of tropic factors and cells in the area of the graft and to provide mechanical support for the newly-formed tissue, but they can be biologically inert. In fact, some of these materials, such as calcium sulphate and calcium phosphate, are slowly bioabsorbable, while others are not reabsorbed, but encapsulated as a foreign body in the natural bone.

Among the various types of paste, prepared with bone tissue available today, it is worth mentioning GRAFTON.RTM^{®}: this is a gel consisting of glycerol (79-85%) and demineralised and lyophilised bone powder (15-21 %), in which the granules have a particle size distribution coming between 0.1 and 1.2 cm. The product is effective for filling small cavities or bone cracks, but it has the drawback of migrating rapidly from the site where it is implanted due to the viscosity and solubility of the glycerol.

WO 2007/056671 A1 discloses a biocompatible hemostatic bone graft containing demineralized bone and a waxy substance, such as polyethylene glycol. Glycerol may be added to the waxy material in some embodiments. Using a demineralized bone matrix in the form of a putty allows the surgeon to mold the material on or near bone, which may enable and promote bone growth.

In the light of the above considerations, it is therefore clearly evident that new materials need to be developed to better satisfy the requirements of operators in the sector, and particularly in the form of pastes that are easy for the surgeon to handle and capable of remaining at the surgical site, enabling the induction of new bone tissue especially when used as a filler in bone cavities and cracks, and in consolidation defects in long bone fractures.

### Summary of the invention

A human bone paste is described that comprising homologous demineralised cortical bone of human origin in the form of a powder, with glycerol and PEG.

### Detailed description of the invention

The present invention enables the above-mentioned problems to be overcome by means of a human bone paste comprising: homologous demineralised cortical bone of human origin in the form of a powder, with glycerol and PEG. Said demineralised powder preferably has a calcium content of less than 5% by weight. According to the invention, the term PEG is used to mean polyethylene glycol having a molecular weight comprised, for example, between 3000 and 6000, and PEG 4000 is particularly preferred, since it is capable of lending the paste sufficient viscosity to make it particularly suitable for use in orthopaedic surgery.

According to the invention, the three above-mentioned components can be contained in the following proportions:

| | |
|---|---|
| Bone powder | 20% /- 40% |
| PEG | 25% /- 45% |
| Glycerol | 25% /- 45% |

The paste according to the invention preferably comprises the following composition:

| | |
|---|---|
| Bone powder | 30% |
| PEG | 35% |
| Glycerol | 35% |

The bone powder is obtained according to known methods, starting with dried cortical bone tissue, by means of a process of tissue pulverisation by means of a bone grinding machine.

The resulting powder undergoes three screenings (lasting 5 minutes, then 2 minutes and 2 minutes), using a vibro-energy mill and suitable screens to obtain a bone powder with a particle size distribution coming between 125µm - 850µm. The bone powder is demineralised by means of repeated treatments thereof, with inorganic acid solutions (sulphuric, hydrochloric, phosphoric). Hydrochloric acid is particularly preferred, in given percentages and for pre-set times. Then the treated powder is rinsed, treated with a phosphate buffer and finally rinsed again.

Four subsequent washes with HCl 0.6N are preferably conducted, keeping the acid in contact with the powder and stirring the mixture for 15 minutes in the first wash, 30 minutes in the second wash, and 2 hours in the last two washes.

Preferably, the proportion of powder to HCI is 1:8 for each cycle.

At the end of each washing cycle, the bone powder is left to settle (with no stirring) and the supernatant is removed by means of a suction pump.

After the last wash with HCI, the powder is rinsed twice in purified water for injections (WFI), preferably in proportions of 1:10 and allowing 15 minutes for each wash.

Then the powder is treated at least twice with a buffer solution at a neutral pH, such as a monobasic phosphate buffer 0.2 M, pH 7.0, preferably in proportions of 1:2, so that the final pH of the powder is comprised between 6.8 and 7.2.

Finally, the powder undergoes two consecutive washes with purified water for 10 minutes each, with the powder and water preferably in proportions of 1:10.

The above-described procedure gives rise to a powder with a calcium content of less than 5%, which is subsequently dried.

The resulting powder is then added to a solution of glycerol and PEG in the above-stated proportions.

The solution of glycerol and PEG is obtained by mixing and warming the two components (preferably to around 70 °C), stirring to obtain a homogeneous solution that is then left to cool to approximately 60 °C. Then the bone powder previously obtained by means of the demineralisation process is added, stirring to obtain a homogeneous paste.

The paste is then distributed in variable doses in special dispensing devices (carpoules) which are sealed under vacuum in a double aluminium/polypropylene bag.

The carpoules are then inserted in a polyurethane foam insulating container, arranged with dry ice between them, and submitted to a sterilising treatment with gamma radiation generated by a Cobalt 60 source. The ionising dose normally used is 60kGy.

The carpoules are then stored at room temperature (15 °C - 30 °C) until use.

The invention is now better explained by means of specific examples that are given below (in the examples, the bone paste according to the invention is also referred to as TBP).

### Example 1

488.9 grams of homologous cortical bone tissue from a human donor were dried in the drier (RETSCH mod. AS100); after 30 minutes at 37 °C, the weight of the tissue amounted to 458.6 grams. Then the dried tissue was ground in the TMI machine (mod. ABG07); after grinding, the resulting 425.0 grams of powder were screened three times in the RETSCH (mod. AS200) vibro-energy mill. After screening, 400.5 grams of powder were obtained (125µm - 850µm).

In the subsequent demineralisation step, the powder was poured into a sterile container, 3.2 L of HCI 0.6N were added (in a ratio of 1:8) and the contents were stirred for 15 minutes. After the established time interval, the agitator was switched off and the powder was left to settle, then the supernatant was removed. Another 3.204 L of HCI 0.6 were added and the same treatment cycle was repeated for 30 minutes. This procedure was repeated twice more, during which cycles the powder was treated with hydrochloric acid for 2 hours.

After the last treatment with hydrochloric acid, the stirring was stopped to allow for the powder to settle, the supernatant was removed and the powder underwent two consecutive washing cycles for 15 minutes each, using 4.9 L of purified water (1:10) for each cycle.

Then, the powder was washed twice more, using for each cycle 0.8 L of monobasic phosphate buffer 0.2M, pH 7.0 (1:2). After the treatments with the buffer, the pH of the solution was 7.1. Finally, the powder was rinsed twice with 4.0 L of purified water (1:10) for 10 minutes.

After the chemical extraction process, the calcium content determined in the powder was 0.4% (colorimetric method - Arsenazo III).

The damp demineralised powder (549.9 grams) was dried (RETSCH drier mod. AS100) for 30 minutes at 37 °C. Since the weight of the dry powder amounted to 295.0 g, the other components of the paste (TBP) were weighed to achieve the following proportions:

| COMPONENTS OF THE TBP | PERCENTAGE (%) | WEIGHT (grams) |
|---|---|---|
| DBM powder | 30 | 295.0 |
| Glycerol | 35 | 342.2 |
| PEG4000 | 35 | 342.2 |

The weighed PEG and glycerol were poured into a container and heated (+70 °C ± 5 °C) while stirring to obtain a homogeneous solution. Then the stirring was stopped and, once the solution had cooled to a temperature of +60 °C, the dried powder was added. The three components were mixed together with a paddle to obtain a homogeneous paste.

At the end of the process, the weight of the TBP was 1,017.4 grams. The paste thus obtained was distributed in 2.5cc, 5.0cc and 10.0cc carpoules.

The filled carpoules were then sealed inside a double bag and submitted to a viral inactivation treatment with gamma radiation.

### Example 2

After 30 minutes at +37 °C (RETSCH drier mod. AS100), 552.4 grams of homologous cortical bone tissue from a human donor were reduced to 520.8 grams.

The dried tissue was ground in the TMI machine (mod. ABG07) and, at the end of the grinding process, the 484.1 grams of powder obtained were screened three times in the RETSCH vibro-energy mill (mod. AS200), giving rise to 461.7 grams of powder (125µm - 850µm) at the end of the screening.

For the demineralisation process, the powder was poured into a sterile container, adding 3.7 L of HCI 0.6N (in a ratio of 1:8) and stirring for 15 minutes. After 15 minutes, the stirrer was switched off and the powder was left to settle, then the supernatant was removed. Then another 3.7 L of HCl 0.6 were added and the treatment cycle was repeated for 30 minutes. This procedure was repeated twice more, during which cycles the powder was treated with hydrochloric acid for 2 hours (1:8).

After the fourth treatment with hydrochloric acid, the stirring was stopped to allow for the powder to settle, the supernatant was removed and the powder underwent two consecutive washing cycles for 15 minutes each, using 4.6 L of purified water (1:10).

The powder was subsequently washed twice more, using 0.9 L of monobasic phosphate buffer 0.2M, pH 7.0 (1:2). At the end of the treatment with the buffer, the pH of the solution was 6.9. Finally, the powder was rinsed twice with 4.6 L of purified water (1:10) for 10 minutes. On completion of the chemical extraction process, the calcium content determined in the powder amounted to 0.7% (colorimetric method - Arsenazo III).

The damp demineralised powder weighing 683.1 grams was dried (RETSCH drier, mod. AS100) for 30 minutes at 37 °C. Since the weight of the dried powder was 278.2 grams, the other components of the TBP were weighed on the basis of the following proportions:

| COMPONENTS OF THE TBP | PERCENTAGE (%) | WEIGHT (grams) |
|---|---|---|
| DBM powder | 30 | 278.2 |
| Glycerol | 35 | 322.7 |
| PEG4000 | 35 | 322.7 |

First the weighed PEG and glycerol were poured into a container and heated (+70 °C ± 5 °C) while stirring to obtain a homogeneous solution. Then the stirring was stopped and, once the solution had cooled to a temperature of +60 °C, the dried powder was added. The three components were mixed together with a paddle to obtain a homogeneous paste. At the end of the process, the weight of the TBP was 911.4 grams. The paste thus obtained was distributed in 2.5cc, 5.0cc and 10.0cc carpoules.

The filled carpoules were then sealed inside a double bag and submitted to a viral inactivation treatment by means of gamma radiation.

### Example 3

583,4 grams of homologous cortical bone tissue from a human donor, obtained starting from 615.7 grams of tissue dried for 30 minutes at +37 °C (RETSCH drier, mod. AS100), were ground in the TMI machine (mod. ABG07). At the end of the grinding process, 545.0 grams of powder were obtained, which were screened three times in the RETSCH vibro-energy mill (mod. AS200), giving rise to 511.7 grams of powder (125µm - 850µm) at the end of the screening.

The screened powder was poured into a sterile container, adding 4.1 L of HCl 0.6N (in a ratio of 1:8) and stirring for 15 minutes. After 15 minutes, the stirrer was switched off and the powder was left to settle, then the supernatant was removed. Then another 4.1 L of HCl 0.6 were added and the treatment cycle was repeated for 30 minutes. This procedure was repeated twice more, during which cycles the powder was treated with hydrochloric acid for 2 hours (1:8).

After the fourth treatment with hydrochloric acid, the stirring was stopped to allow for the powder to settle, the supernatant was removed and the powder underwent two consecutive washing cycles for 15 minutes each, using 5.1 L of purified water (1:10).

Then, the powder was washed twice more with 1.0 L of monobasic phosphate buffer 0.2M, pH 7.0 (1:2). At the end of the treatment with the buffer, the pH of the solution was 6.9. Then the powder was rinsed twice with 5.1 L of purified water (1:10) for 10 minutes. On completion of the chemical extraction process, the calcium content determined in the powder amounted to 1.4% (colorimetric method - Arsenazo III).

The damp demineralised powder weighing 683.8 grams was dried (RETSCH drier, mod. AS100) for 30 minutes at 37 °C. Since the weight of the dried powder was 295.1 grams, the other components of the TBP were weighed on the basis of the following proportions:

| COMPONENTS OF THE TBP | PERCENTAGE (%) | WEIGHT (grams) |
|---|---|---|
| DBM powder | 30 | 295.1 |
| Glycerol | 35 | 342.3 |
| PEG4000 | 35 | 342.3 |

The PEG and glycerol were heated (+70 °C±5 °C) and stirred to obtain a homogeneous solution; then the stirring was stopped and, once the solution had cooled to a temperature of +60 °C, the dried powder was added. The three components were mixed together with a paddle to obtain a homogeneous paste. At the end of the process, the weight of the TBP was 911.4 grams. The paste thus obtained was distributed in 2.5cc, 5.0cc and 10.0cc carpoules.

The filled carpoules were then sealed inside a double bag and submitted to a viral inactivation treatment by means of gamma radiation.

Biological tests conducted to assess the osteo-inductive capacity of the resulting product, based on the monitoring of markers such as alkaline phosphatase activity and the concentration of osteocalcin in stem cell cultures, confirmed the non-toxicity and the osteo-inductive capacity of the paste. The alkaline phosphatase activity, measured on samples in various stages of the paste production process, demonstrated that the osteogenic capacity of the end product is not significantly lower than that of the original product at the baseline, as confirmed by the excerpt of the results given below.

### Alkaline phosphatase assay

| **SAMPLE (15 g)** | **SPECIFIC ACTIVITY (Unit/mg protein)** |
|---|---|
| Positive control | 681 |
| Powder | 722 |
| Demineralised powder | 625 |
| Gamma irradiated demineralised powder | 584 |
| Gamma-irradiated TBP | 801 |

Osteocalcin (a marker of ossification and consequently of osteogenesis) was also assayed, confirming the results obtained for alkaline phosphatase activity: in fact, no significant reduction in the osteo-inductive potential of the product was detectable during the paste production process:

### Osteocalcin assay

| **SAMPLE** | **CONCENTRATION (ng/mg protein)** |
|---|---|
| Positive control | 31.0 |
| Powder | 22.7 |
| Demineralised powder | 16.1 |
| Gamma irradiated demineralised powder | 17.5 |
| Gamma-irradiated TBP | 16.8 |

## Claims

1. A human bone paste, sterilised and virus inactivated by means of gamma radiation, comprising: homologous demineralised cortical bone of human origin in powdered form having a particle size distribution comprised between 125µm and 850µm, with glycerol and PEG 4000; said paste having the following percentage composition:
| | |
|---|---|
| Bone powder | 20% /- 40% |
| PEG | 25% /- 45% |
| Glycerol | 25% /- 45%; |
wherein said demineralised bone powder has a calcium content of less than 5% by weight and said PEG is PEG 4000.

2. A paste according to claim 1, containing the following percentage composition:
| | |
|---|---|
| Bone powder | 30% |
| PEG | 35% |
| Glycerol | 35% |

3. A process for the preparation of a paste according to claims 1 or 2, wherein:
- a bone powder is obtained starting from dried cortical bone tissue by means of a process of pulverisation of the tissue in a bone grinding machine and subsequent screening to obtain a particle size distribution comprised between 125µm and 850µm;
- the powder is demineralised, washed with a phosphate buffer and purified water and then dried;
- the resulting powder is added to a solution of glycerol and PEG 4000, mixing to obtain a homogeneous paste that is cooled and then distributed in carpoules that are sealed under a vacuum in a double aluminium/polypropylene bag and submitted to a gamma ray sterilising and virus inactivating treatment.
wherein said demineralisation is achieved in the following steps:
- the bone powder obtained from the first steps in the process undergoes 4 washing cycles with HCl 0.6N, in proportions of 1:8, leaving the acid in contact with the powder for 15 minutes in the first wash, 30 minutes in the second, and 2 hours in the third and fourth;
- the powder is then washed twice with purified water in proportions of 1:10, each washing cycle lasting 15 minutes;
wherein the solution of glycerol and PEG is obtained by mixing the two components at approximately 70 °C while stirring in order to obtain a homogeneous solution that is then left to cool to approximately 60 °C before adding the demineralised powder.

4. The paste according to claims 1 to 2 for use in bone repair and reconstruction procedures.

## Patentansprüche

1. Humane Knochenpaste, welche mittels Gammastrahlung sterilisiert und virusinaktiviert ist, und welche enthält: homologen demineralisierten kortikalen Knochen menschlichen Ursprungs in Pulverform mit einer Partikelgrößenverteilung zwischen 125 µm und 850 µm zusammen mit Glycerin und PEG 4000, wobei die Paste die nachfolgende prozentuale Zusammensetzung aufweist:
| | |
|---|---|
| Knochenpulver | 20 % - 40 % |
| PEG | 25 % - 45 % und |
| Glycerin | 25 % - 45 %, |
wobei das demineralisierte Knochenpulver einen Calciumgehalt von weniger als 5 Gew.-% aufweist und das PEG PEG 4000 ist.

2. Paste nach Anspruch 1, welche die nachfolgende prozentuale Zusammensetzung aufweist:
| | |
|---|---|
| Knochenpulver | 30 % |
| PEG | 35 % und |
| Glycerin | 35 %. |

3. Verfahren zum Herstellen einer Paste nach Anspruch 1 oder 2, wobei:
- ein Knochenpulver ausgehend von getrocknetem kortikalem Knochengewebe mittels eines Verfahrens des Pulverisierens des Gewebes in einer Knochenmahlmaschine und nachfolgenden Siebens, um eine Partikelgrößenverteilung zwischen 125 µm und 850 µm zu erhalten, erhalten wird,
- das Pulver demineralisiert wird, mit einem Phosphatpuffer gewaschen wird und mit Wasser gereinigt wird und dann getrocknet wird,
- das resultierende Pulver zu einer Lösung von Glycerin und PEG 4000 zugegeben wird und vermischt wird, um eine homogene Paste zu erhalten, welche abgekühlt wird und dann in Flaschen verteilt wird, welche unter einem Vakuum in einen doppelten Aluminium-/ Polypropylen-Beutel eingeschweißt werden und einer Gammastrahlensterilisierung und einer Virusinaktivierungsbehandlung unterzogen werden,
wobei die Demineralisierung durch die nachfolgenden Schritte erreicht wird:
- das aus dem ersten Schritt in dem Verfahren erhaltene Knochenpulver unterläuft vier Waschzyklen mit 0,6 N HCl in Verhältnissen von 1:8, wodurch die Säure in Kontakt mit dem Pulver für 15 Minuten in der ersten Wäsche belassen wird, für 30 Minuten in der zweiten Wäsche belassen wird und für 2 Stunden in der dritten und vierten Wäsche belassen wird,
- das Pulver wird dann zweimal mit gereinigtem Wasser in Verhältnissen von 1:10 gewaschen, wobei jeder Waschzyklus wenigstens 15 Minuten andauert,
wobei die Lösung aus Glycerin und PEG durch Mischen der beiden Komponenten bei ungefähr 70°C unter Rühren erhalten wird, um eine homogene Lösung zu erhalten, welche dann belassen wird, um auf ungefähr 60°C vor der Zugabe des demineralisierten Pulvers abzukühlen.

4. Paste nach einem der Ansprüche 1 bis 2 zur Verwendung in der Knochenreparatur und in Rekonstruktionsverfahren.

## Revendications

1. Pâte osseuse humaine, stérilisée et inactivée quant au virus au moyen d'un rayonnement gamma, comprenant:
un os cortical homologue déminéralisé d'origine humaine sous forme de poudre ayant une distribution de dimensions des particules comprise entre 125µm et 850µm, avec du glycérol et PEG 4000; ladite pâte ayant la composition suivante en pourcentage:
| | |
|---|---|
| Poudre d'os | 20% - 40% |
| PEG | 25% - 45% |
| Glycérol | 25% - 45%; |
où ladite poudre d'os déminéralisé a une teneur en calcium inférieure à 5% en poids, et ledit PEG est le PEG 4000.

2. Pâte selon la revendication 1, contenant la composition suivante en pourcentage:
| | |
|---|---|
| Poudre d'os | 30% |
| PEG | 35% |
| Glycérol | 35% |

3. Procédé de préparation d'une pâte selon les revendications 1 ou 2, dans lequel:
- une poudre d'os est obtenue à partir d'un tissu osseux cortical séché au moyen d'un processus de pulvérisation du tissu dans une machine pour broyer l'os et ensuite le criblage pour obtenir une distribution des dimensions des particules comprise entre 125µm et 850µm;
- la poudre est déminéralisée, lavée avec un tampon de phosphate et de l'eau purifiée et est ensuite séchée;
- la poudre obtenue est ajoutée à une solution de glycérol et de PEG 4000, mélangée pour obtenir une pâte homogène qui est refroidie et ensuite distribuée en capsules qui sont scellées sous vide dans un sachet d'aluminium/polypropylène double et soumises à un traitement de stérilisation aux rayons gamma et d'inactivation de virus,
où ladite déminéralisation est atteinte par les étapes suivantes:
- la poudre d'os obtenue par les premières étapes dans le processus est soumise à quatre cycles de lavage avec HCl 0,6N, dans des proportions de 1:8, en laissant l'acide en contact avec la poudre pendant 15 minutes lors du premier lavage, 30 minutes lors du deuxième et 2 heures lors des troisième et quatrième;
- la poudre est ensuite lavée deux fois avec de l'eau purifiée dans les proportions de 1:10, chaque cycle de lavage durant 15 minutes;
où la solution de glycérol et de PEG est obtenue en mélangeant les deux composants environ à 70°C tout en agitant pour obtenir une solution homogène qu'on laisse ensuite refroidir à environ 60°C avant d'ajouter la poudre déminéralisée.

4. Pâte selon les revendications 1 à 2 pour utilisation dans des procédures de réparation et de reconstruction osseuse.
